# EUROPEAN PATENT APPLICATION

(11) **EP 1 997 880 A1**
(43) Date of publication of application: **03.12.2008**
(21) Application number: 07380157.3
(22) Date of filing: 31.05.2007
(51) Int. Cl.: C12N 1/20, C12Q 1/24, A23L 1/03, A61K 35/74, C12R 1/01

(54) **Breast milk bifidobacteria, compositions thereof, their use and a novel culture media to obtain them**

(71) Applicant: PULEVA BIOTECH, S.A., 18004 Granada (ES)
(72) Inventor: Olivares Martin, Mónica, 18198 Huetor Vega Granada (ES); Jiménez Quintana, Esther Antonia, 28460 Los Molinos Madrid (ES); Sierra Avila, Saleta, 18005 Granada (ES); Marin Martinez, Maria Luisa, 28002 Madrid (ES); Lara Villoslada, Federico, 18220 Albolote Granada (ES); Arroyo Rodriguez, Rebeca, 28850 Torrejón de Ardoz Madrid (ES); Martin Jiménez, Rocio, 11202 Algeciras Cádiz (ES); Maldonado Barragán, Antonio, 41100 Coria del Rio Sevilla (ES); Martin Merino, Virginia, 28028 Madrid (ES); Boza Puerta, Julio, 18008 Granada (ES); Jiménez López, Jesús, 18110 Granada (ES)
(74) Representative: ABG Patentes, S.L.

(57) **Abstract**

The present invention relates to new probiotic *Bifidobacterium* strains, obtained from breast milk by means of a new culture media, and their use for the prophylaxis or treatment against digestive, infective, neuro-degenerative and other immune related diseases such as allergies or inflammatory diseases, and to compositions comprising these compounds.

SEQUENCE LISTING

<110> PULEVA BIOTECH

<120> BREAST MILK BIFIDOBACTERIA, COMPOSITIONS THEREOF, THEIR USE AND A
NOVEL CULTURE MEDIA TO OBTAIN THEM

<130> P2903EP00

<160> 2

<170> PatentIn version 3.4

<210> 1
<211> 481
<212> DNA
<213> Bifidobacterium breve CECTXXXX

<220>
<221> misc_feature
<222> (4)..(4)
<223> n is a, c, g, or t

<220>
<221> misc_feature
<222> (7)..(8)
<223> n is a, c, g, or t

<220>
<221> misc_feature
<222> (13)..(13)
<223> n is a, c, g, or t

<220>
<221> misc_feature
<222> (25)..(25)
<223> n is a, c, g, or t

<220>
<221> misc_feature
<222> (131)..(131)
<223> n is a, c, g, or t

<220>
<221> misc_feature
<222> (147)..(147)
<223> n is a, c, g, or t

<400> 1
tcgngtnnga agnacaataa acacntaagt gccttgctcc ctaacaaaag aggtttacaa 60

cccgcaaggc ctccatccct cacgcggcgt cgctgcatca ggcttgcgcc cattgatgca 120

atattcccca ntgctgcctc ccgatangag tctgggccgt atctcagtcc caatgtggcc 180

ggtcgccctc tcaggccggc tacccgtcga agccatggtg ggccgttacc ccgccatcaa 240

gctgatagga cgcgacccca tcccatgccg caaaggcttt cccaacacac catgcggtgt 300

gatggagcat ccggcattac cacccgtttc caggagctat tccggtgcat ggggcaggtc 360

ggtcacgcat tactcacccg ttcgccactc tcaccaccag gcaaagcccg atggatcccg 420

ttcgacttgc atgtgttaag cacgccgcca gcgttcatcc tgagccagga tcaaactcta 480

a 481

<210>  2
<211>  476
<212>  DNA
<213>  Brevibacterium breve CECTXXXX


<220>
<221>  misc_feature
<222>  (2)..(2)
<223>  n is a, c, g, or t

<220>
<221>  misc_feature
<222>  (8)..(8)
<223>  n is a, c, g, or t

<220>
<221>  misc_feature
<222>  (63)..(63)
<223>  n is a, c, g, or t

<220>
<221>  misc_feature
<222>  (72)..(72)
<223>  n is a, c, g, or t

<220>
<221>  misc_feature
<222>  (80)..(80)
<223>  n is a, c, g, or t

<220>
<221>  misc_feature
<222>  (83)..(83)
<223>  n is a, c, g, or t

<220>
<221>  misc_feature
<222>  (87)..(87)
<223>  n is a, c, g, or t

<220>
<221>  misc_feature
<222>  (114)..(114)
<223>  n is a, c, g, or t

<220>
<221>  misc_feature
<222>  (141)..(141)
<223>  n is a, c, g, or t

<220>
<221>  misc_feature
<222>  (159)..(159)
<223>  n is a, c, g, or t

<220>
<221>  misc_feature
<222>  (161)..(161)
<223>  n is a, c, g, or t

<220>
<221>  misc_feature
<222>  (355)..(355)
<223>  n is a, c, g, or t

<220>

<221>  misc_feature
<222>  (358)..(358)
<223>  n is a, c, g, or t

<220>
<221>  misc_feature
<222>  (369)..(369)
<223>  n is a, c, g, or t

<220>
<221>  misc_feature
<222>  (444)..(444)
<223>  n is a, c, g, or t

<220>
<221>  misc_feature
<222>  (455)..(456)
<223>  n is a, c, g, or t

<400>  2
tncgcganga agaaataaaa caaagtgcct tgctccctaa caaaagaggt ttacaacccg      60

aangcctcca tncctcacgn ggngtcncat gcatcaggct tgcgcccatt gtgnaatatt     120

ccccactgct gcctcccgta ngagtctggg ccgtatctna ntcccaatgt ggccggtcgc     180

cctctcaggc cggctacccg tcgaagccat ggtgggccgt taccccgcca tcaagctgat     240

aggacgcgac cccatcccat gccgcaaagg ctttcccaac acaccatgcg gtgtgatgga     300

gcatccggca ttaccacccg tttccaggag ctattccggt gcatggggca ggtcngtnac     360

gcattactna cccgttcgcc actctcacca ccaggcaaag cccgatggat cccgttcgac     420

ttgcatgtgt taagcacgcc gccngcgttc atccnnaaac aggatcaaac tctaaa         476

SEQUENCE LISTING

<110>  PULEVA BIOTECH

<120>  BREAST MILK BIFIDOBACTERIA, COMPOSITIONS THEREOF, THEIR USE AND A
        NOVEL CULTURE MEDIA TO OBTAIN THEM

<130>  P2903EP00

<160>  2

<170>  PatentIn version 3.4

<210>  1
<211>  481
<212>  DNA
<213>  Bifidobacterium breve CECT7263

<220>
<221>  misc_feature
<222>  (4)..(4)
<223>  n is a, c, g, or t

<220>
<221>  misc_feature
<222>  (7)..(8)
<223>  n is a, c, g, or t

<220>
<221>  misc_feature
<222>  (13)..(13)
<223>  n is a, c, g, or t

<220>
<221>  misc_feature
<222>  (25)..(25)
<223>  n is a, c, g, or t

<220>
<221>  misc_feature
<222>  (131)..(131)
<223>  n is a, c, g, or t

<220>
<221> misc_feature
<222> (147)..(147)
<223> n is a, c, g, or t

<400> 1
tcgngtnnga agnacaataa acacntaagt gccttgctcc ctaacaaaag aggtttacaa 60

cccgcaaggc ctccatccct cacgcggcgt cgctgcatca ggcttgcgcc cattgatgca 120

atattcccca ntgctgcctc ccgatangag tctgggccgt atctcagtcc caatgtggcc 180

ggtcgccctc tcaggccggc tacccgtcga agccatggtg ggccgttacc ccgccatcaa 240

gctgatagga cgcgacccca tcccatgccg caaaggcttt cccaacacac catgcggtgt 300

gatggagcat ccggcattac cacccgtttc caggagctat tccggtgcat ggggcaggtc 360

ggtcacgcat tactcacccg ttcgccactc tcaccaccag gcaaagcccg atggatcccg 420

ttcgacttgc atgtgttaag cacgccgcca gcgttcatcc tgagccagga tcaaactcta 480

a 481

<210> 2
<211> 476
<212> DNA
<213> Brevibacterium breve CECT7264

<220>
<221> misc_feature
<222> (2)..(2)
<223> n is a, c, g, or t

<220>
<221> misc_feature
<222> (8)..(8)
<223> n is a, c, g, or t

<220>
<221> misc_feature
<222> (63)..(63)

<223>  n is a, c, g, or t

<220>
<221>  misc_feature
<222>  (72)..(72)
<223>  n is a, c, g, or t

<220>
<221>  misc_feature
<222>  (80)..(80)
<223>  n is a, c, g, or t

<220>
<221>  misc_feature
<222>  (83)..(83)
<223>  n is a, c, g, or t

<220>
<221>  misc_feature
<222>  (87)..(87)
<223>  n is a, c, g, or t

<220>
<221>  misc_feature
<222>  (114)..(114)
<223>  n is a, c, g, or t

<220>
<221>  misc_feature
<222>  (141)..(141)
<223>  n is a, c, g, or t

<220>
<221>  misc_feature
<222>  (159)..(159)
<223>  n is a, c, g, or t

<220>
<221>  misc_feature
<222>  (161)..(161)
<223>  n is a, c, g, or t

<220>
<221>  misc_feature

<222>  (355)..(355)
<223>  n is a, c, g, or t

<220>
<221>  misc_feature
<222>  (358)..(358)
<223>  n is a, c, g, or t

<220>
<221>  misc_feature
<222>  (369)..(369)
<223>  n is a, c, g, or t

<220>
<221>  misc_feature
<222>  (444)..(444)
<223>  n is a, c, g, or t

<220>
<221>  misc_feature
<222>  (455)..(456)
<223>  n is a, c, g, or t

<400>  2
tncgcganga agaaataaaa caaagtgcct tgctccctaa caaaagaggt ttacaacccg      60

aangcctcca tncctcacgn ggngtcncat gcatcaggct tgcgcccatt gtgnaatatt     120

ccccactgct gcctcccgta ngagtctggg ccgtatctna ntcccaatgt ggccggtcgc     180

cctctcaggc cggctacccg tcgaagccat ggtgggccgt taccccgcca tcaagctgat     240

aggacgcgac cccatcccat gccgcaaagg ctttcccaac acaccatgcg gtgtgatgga     300

gcatccggca ttaccacccg tttccaggag ctattccggt gcatggggca ggtcngtnac     360

gcattactna cccgttcgcc actctcacca ccaggcaaag cccgatggat cccgttcgac     420

ttgcatgtgt taagcacgcc gccngcgttc atccnnaaac aggatcaaac tctaaa         476

## Description

### FIELD OF THE INVENTION

The present invention relates to a novel media and selection conditions for the isolation of probiotic strains from human breast milk of the genus Bifidobacterium as well as to the strains isolated and the uses thereof for the prophylaxis and treatment against digestive, infective, neurodegenerative and immune related diseases such as allergies or inflammatory diseases.

### BACKGROUND OF THE INVENTION

For many years, lactic acid bacteria have been utilized as fermenting agents for the preservation of food taking benefit of a low pH and the action of fermentation products generated during the fermentative activity thereof to inhibit the growth of spoilage bacteria. With this aim, not well characterized lactic acid bacteria or "fermentum" have been used to prepare a variety of different foodstuffs such as dry fermented meat products, cheese, and other fermented dairy products from milk.

Recently, lactic acid bacteria have attracted a great deal of attention because some strains have been found to exhibit valuable properties to man and animals upon ingestion. In particular, specific strains of the genus *Lactobacillus* or *Bifidobacterium* have been found to be able to colonize the intestinal mucosa and to assist in the maintenance of the well-being of man and animal. Moreover, it has been demonstrated that the initial colonization of the neonatal gut with such bacteria is a crucial factor for the correct maturation and development of the neonatal immune system. For all these reasons, the consumption of these bacteria, currently known as probiotics, is in fact an attractive approach for the improvement of the human well-being and health.

Probiotics are considered to be viable microbial preparations which promote the individual's health by preserving a healthier microflora in the intestine. A microbial preparation may be commonly accepted as a probiotic in case the effect thereof and their mode of action are known. Probiotics are deemed to attach to the intestine's mucosa, colonize the intestinal tract and likewise prevent attachment of harmful microorganisms thereon. A crucial prerequisite for their action resides in that they have to reach the gut's mucosa in a proper and viable form and do not get destroyed in the upper part of the gastrointestinal tract, especially by the influence of the low pH prevailing in the stomach.

In this regard, several patent applications such as e.g. EP0768375, WO97/00078, EP0577903 and WO00/53200 disclose specific strains of *Bifidobacterium* and *Lactobacillus* and their beneficial effects on diarrhea, immunomodulation, hypersensitivity reactions or infection by pathogen microorganisms.

The international patent application WO2004/003235 describes the selection and use of several Lactobacilli strains present in human breast milk that could confer health benefits for humans after their consumption. From that time, several works have described the microbiota of human breast milk and postulated the mechanism used for these bacteria in order to be transferred from mother's gut to the mammary gland. The main cultured strains described in this works are members of the *Lactobacillus, Enterococcus, Streptococcus* and *Staphylococcus* genera. However, new molecular studies have suggested the presence of at least DNA from bifidobacteria, although it was not possible to never obtain them from breast milk due to the extreme anaerobiosis and growth factor dependency of these bacteria to grow.

In view of the valuable properties that particular strains of lactic acid bacteria may provide, there is a desire in the art for additional lactic acid bacterial strains that are beneficial to the well being of man and/or animal. Consequently, there is a need to provide rational methods and sources which allow obtaining bacterial strains which may exhibit beneficial properties for man and/or animals.

### SUMMARY OF THE INVENTION

In a first aspect, the invention provides a lactic acid bacteria culture media supplemented with olive oil,_fish oil, ascorbic acid, rice bran extract, fructo-oligosaccharides and goat casein.

In a further aspect, the invention provides the use of the culture media of the invention as a media to grow probiotic bacteria.

In a further aspect, the invention provides a method for the isolation of probiotic bacteria comprising the steps of
(i) Incubating a sample suspected of containing a probiotic strain in the culture medium of the invention and
(ii) selecting strains which are capable of growing in said culture medium.

In a further aspect, the invention further provides a bacterium obtainable by the method of the invention.

In a further aspect, the bacteria is selected from the group of the bacteria deposited in the CECT under Accession N° 7263 and a bacteria deposited in the CECT under Accession N° 7264.

In another aspect, the invention provides a composition comprising at least one of the bacterial strains of the invention. In a preferred embodiment, the composition comprises another strain or mixture of strains.

In another aspect, the invention provides a foodstuff or animal feed product comprising at least one of the strains of the invention.

In another aspect, the invention provides a pharmaceutical product comprising a therapeutically effective dose of at least one of the strains of the invention.

In a further aspect, the invention provides a bacterial strain according of the invention or a composition of the invention for therapeutic or prophylactic treatment of human and animal diseases.

In a further aspect, the invention provides for the use of a strain of the invention or a compositions of the invention for the manufacture of a medicament for the therapeutic or prophylactic treatment of chronic or acute infection or of undesirable microbial colonization of any body surface or mucosa in a subject or animal in need thereof.

In a further aspect, the invention provides the use of a strain or of a composition according to the invention in the manufacture of a medicament for the treatment and/or prophylaxis of temporally depressed immune levels in a subject or animal in need thereof.

In a further aspect, the invention provides the use of a strain or of a composition according to the invention in the manufacture of a medicament for the improvement of the immune gut barrier in a subject or animal in need thereof; for the treatment and/or prophylaxis of down-regulating hypersensitivity reactions to food and metabolic intolerance such as lactose intolerance; of constipation and other gastro-intestinal disorders; of inflammatory or auto-immune disorders such as: IBD, ulcerative colitis, arthritis, atherosclerosis, multiple sclerosis, psoriasis or sarcoidosis; and of tumor growth, metastasis and cancer in subject or animal in need thereof.

In a further aspect, the invention provides the use of a strain or of a composition according to the invention in the manufacture of a medicament for the treatment and/or prophylaxis of allergic disorders and asthma in a subject or animal in need thereof.

In a further aspect, the invention provides the use of a strain or of a composition according to the invention in the manufacture of a medicament for the treatment and/or prophylaxis of neurodegenerative diseases in an individual or animal in need thereof.

In a further aspect, the invention provides the use of a strain or of a composition according to the invention in the manufacture of a medicament for the treatment and/or prophylaxis of metabolic and body weight control disorders.

In a further aspect, the invention provides the use of a strain or of a composition according to the invention in the manufacture designed to be administered to lactating woman for the therapeutic or prophylactic treatment of their fetus and/or their breastfed babies.

### BRIEF DESCRIPTION OF THE FIGURES

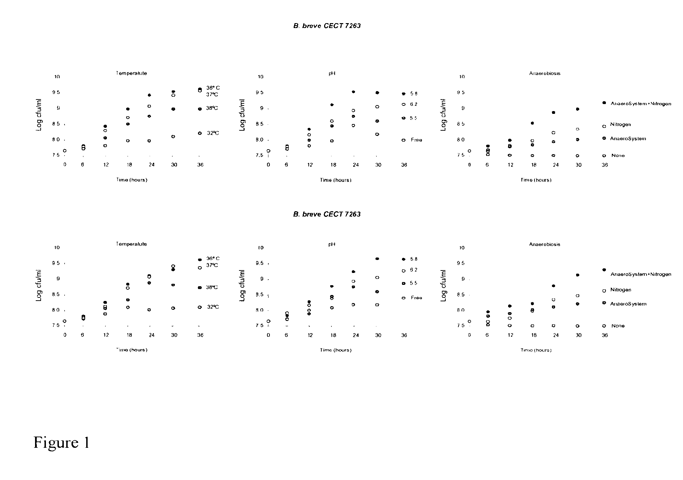

Figure 1 illustrates the growing curves of probiotic strains in different culture media. Similar amount of bifidobacteria strains were inoculated in different media, and the amount of viable bacteria counts were analyzed at 6 hour intervals during 30 hours.

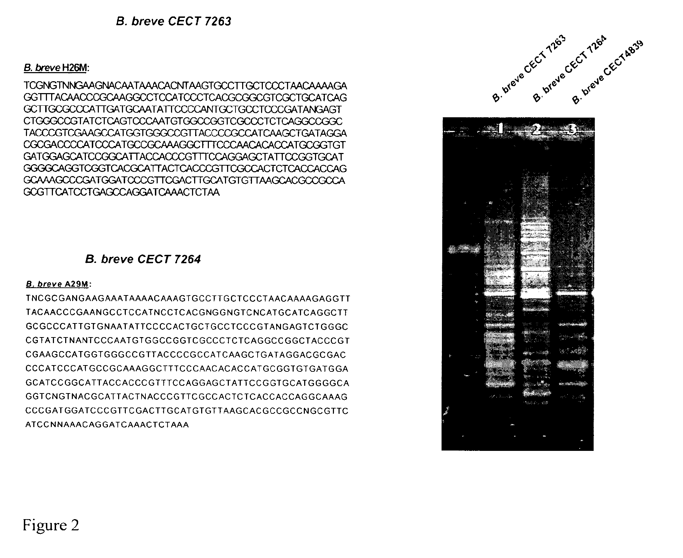

Figure 2 illustrates the 16S rRNA sequence of both bacteria included in this invention *(B. breve* CECT7263 and CECT7264) and their PCR-RAPD profile.

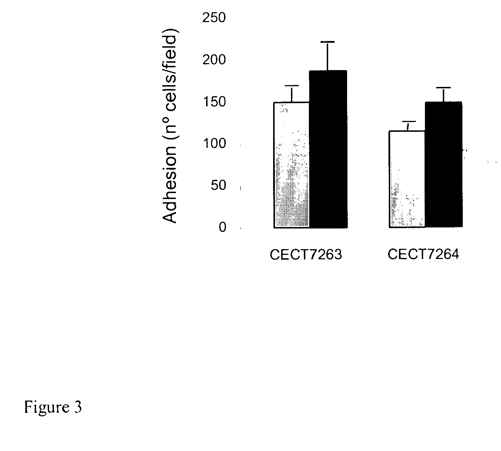

Figure 3 illustrates the adhesion of probiotic strains to intestinal cells. The adhesion of the probiotic strains of this invention were assessed using Caco-2 (grey bars) or HT-29 (black bars) intestinal cell lines. Twenty randomized fields were counted and the results expressed as the mean of the number of bacteria attached to the cells per field ±SD.

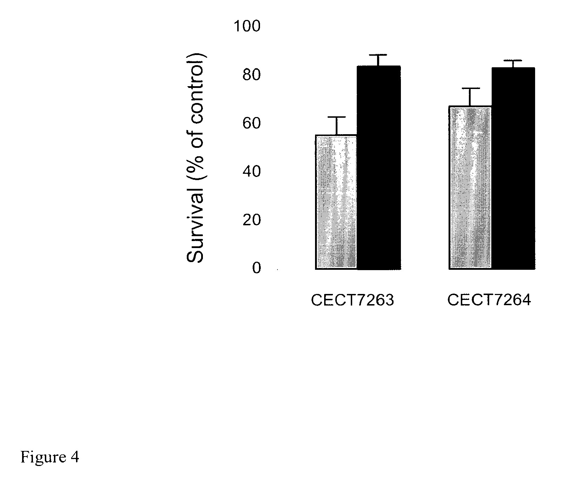

Figure 4 illustrates the survival of probiotic strains to digestion conditions. The resistance of the probiotic strains of this invention to acidic (grey bars) and high bile salt content (black bars) was assessed in vitro by culture of bacteria in MRS pH 3.0 or 0.15% bile salts for 90 minutes. The results are represented as the mean ±SD of three independent experiments.

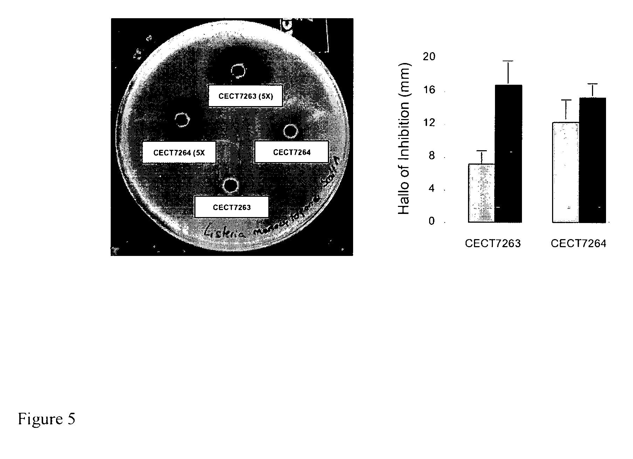

Figure 5 illustrates the production of antimicrobial metabolites by the probiotic strains. The production of bactericide metabolites by the probiotic strains of this invention was assessed *in vitro* by an agar well diffusion assay in TSA plates cultured with *Listeria monocitogenes* (grey bars) or *Salmonella typhimurium* (black bars). The diameter of the hallo (in millimeters) of inhibition induced by the bacterial supernatants determines the bactericide effect.

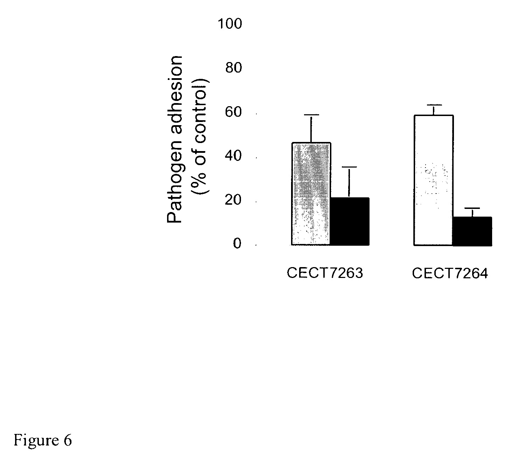

Figure 6 illustrates the inhibition of the adhesion of pathogenic bacteria. The adhesion of the pathogenic strains *E. coli* (grey bars) and *S. typhimurium* (black box) to Caco-2 cells was assessed in the presence of the probiotic strains of this invention and compared to commercial probiotic strains. Ten randomized fields were counted and the results expressed as the mean of the % of adhered gram-negative bacteria attached to the cells compared to the number of pathogenic bacteria adhered in absence of probiotics.

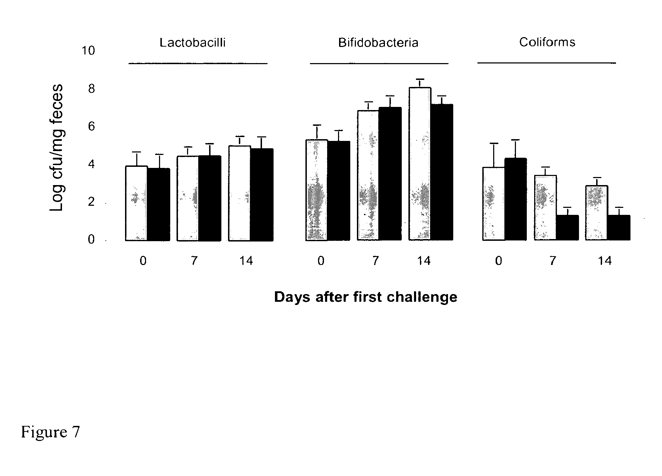

Figure 7 illustrates the gut colonization by *B. breve* CECT 7263 (grey bars) and CECT 7264 (black bars). The number of fecal lactobacillus, bifidobacteria and coliform bacteria in mice supplemented daily for 14 days with 10⁸ cfu of *B. breve* CECT 7263 and CECT 7264 was analyzed by bacterial platting. Fecal samples (ca. 200 mg) were collected at day 0, 7 and 14 of probiotic supplementation.

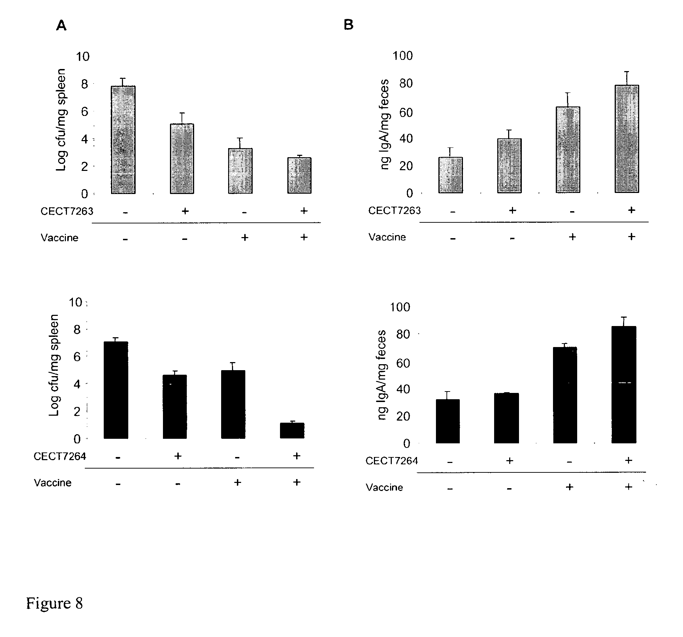

Figure 8 illustrates the effect of *B. breve* CECT 7263 and CECT 7264 on *Salmonella* infection. A) *B. breve* CECT 7263 and CECT 7264 inhibit *Salmonella* translocation to the spleen. The number of Salmonella colonies was measured in the spleens of mice treated with *bifidobacteria* with or without vaccination with 10⁸ inactivated cfu of *Salmonella* after 24 hour of an oral challenge with 10¹⁰ cfu *Salmonella.* B) The same mice were used to measure the IgA content in feces.

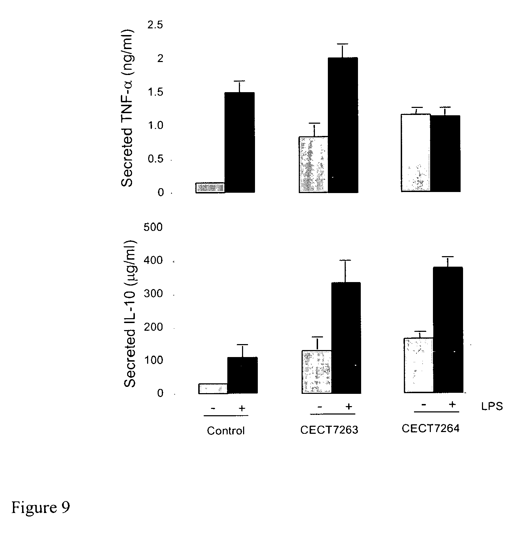

Figure 9 illustrates the effect of probiotic strains on cytokine expression. The TNF-α (A) or IL-10 (B) cytokine production was analyzed in bone marrow derived macrophages stimulated with LPS and the indicated probiotic strain for 12 hours. Cytokine production was detected by an ELISA technique.

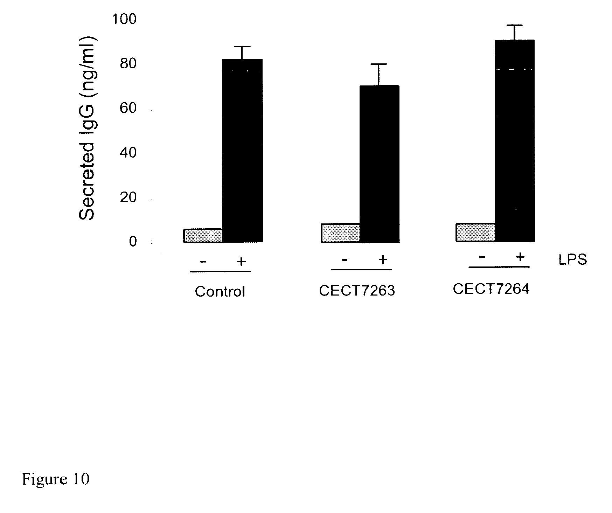

Finally, figure 10 illustrates the effect of probiotic strains on Ig G expression. The IgG production was analyzed in lymphocytes obtained from the spleen of Balb/c mice (6-8 weeks old) stimulated with LPS and the indicated probiotic strain for 6 days. Immunoglobulin production was detected by an ELISA technique from Bethyl Laboratories.

### DETAILED DESCRIPTION OF THE INVENTION

The inventors have surprisingly identified culture medium and conditions in which different strains of the genus Bifidobacterium present in human breast milk can be isolated. Accordingly, in a first aspect, the invention provides a culture medium which comprises a lactic acid bacteria culture medium supplemented with olive oil, fish oil, ascorbic acid, rice bran extract, fructo-oligosaccharides and goat casein.

Any conventional lactic acid bacteria culture media known in the art can be used in the present invention as long as it is supplemented with the above components. Media suitable for use in the present invention are, for instance, MRS medium, APT medium. RCM medium, LM 17 medium, GM17 medium and Elliker medium.

"Olive oil" refers to the vegetable oil obtained from the olive (*Olea europaea*)*.* Any retail grade is suitable for use in the present invention including extra-virgin olive oil, virgin olive oil, olive oil, olive-pomace, lampante oil and mixtures thereof.

"Fish oil" refers to any oil isolated from any fish. Preferably, the oils which form part of the invention have a total n-3 fatty acid content of greater, than about 20% such as are derived from menhaden oil, herring, capelin, anchovy, cod liver, salmon oil, tuna oil, sardine oil and mixtures thereof.

"Ascorbic acid" refers to (*R*)-3,4-dihydroxy-5-((*S*)- 1,2-dihydroxyethyl)furan-2(5*H*)-one, the compound whose L-entantiomer is commonly known as vitamin C.

"Rice bran extract" refers to an extract of the outer layer of brown rice. The processes of this invention preferably use raw rice bran. However, dried rice bran, stabilized rice bran or defatted rice bran may also be extracted. The extract can be prepared by any extraction means known in the art.

"Fructo-oligosaccharides" (FOS) refer to short-chain oligosaccharides comprised of D-fructose and D-glucose, containing from 3 to 5 monosaccharide units. FOS, also called neosugar and short-chain FOS (scFOS), are produced on a commercial scale from sucrose using a fungal fructosyltransferase enzyme and are comprised of one molecule of D-glucose in the terminal position and from 2 to 4 D-fructose units. Preferred FOS for use in the present invention are those containing 2 fructose residues (1-kestose or GF₂), 3 fructoses (GF₃ or nystose), 4 fructose residues (GF4 or 1-β-fructo-furanosyl nystose) or mixtures thereof.

"Goat casein" refers to the micelles isolated from goat milk comprising one or more of the casein variants including alpha S1 casein (Uniprot P18626), alpha S2 casein (Uniprot P33049,), beta casein (Uniprot P33049) and kappa casein (Uniprot P02670).

In a preferred embodiment, the culture medium of the invention comprises peptone, goat casein, glucose, yeast extract, Tween 80, sodium acetate, magnesium sulfate, tri-ammonium citrate, olive oil, fish oil, rice bran extract, fructo-oligosaccharides, dicloxacilin, lithium chloride, ascorbic acid and L-cysteine hydrochloride.

The culture media may contain any concentration of each of those components. Preferably, the concentrations of the different components are 0.01% to 1000%, more preferably from 0.1 % to 100%, and even more preferably from 10% to 50%.

In a more preferred embodiment, the culture medium is the M56 medium, which compositions is as defined in Table 1.

**Table 1. Composition of the M56 medium**

| **Component** | **Concentration (g/l)** |
|---|---|
| Peptone | 10 |
| Goat casein | 5 |
| Glucose | 15 |
| Yeast extract | 2 |
| Tween 80 | 2 |
| Sodium acetate | 5 |
| Magnesium sulphate | 0.2 |
| Tri-amonium citrate | 2 |
| Olive oil | 2 |
| Fish oil | 0.5 |
| Rice bran extract | 1 |
| Fructo-oligosaccharides | 5 |
| Dicloxacilin | 0.0005 |
| Lithium chloride | 1 |
| Ascorbic acid | 0.5 |
| L-cysteine hydrochloride | 0.5 |

The medium of the invention has proved to be particularly suitable for the growth of lactic acid bacteria, particularly for bacteria of the genus Bifidobacterium. Accordingly, in a further aspect, the invention provides the use of the culture media of the invention to grow probiotic bacteria.

In a further aspect, the invention provides a method for the isolation of probiotic bacteria comprising the steps of
(i) incubating a sample suspected of containing a probiotic strain in the culture medium of the invention and
(ii) selecting strains which are capable of growing in said culture medium.

In a preferred embodiment, the sample suspected of containing a probiotic strain is human breast milk.

In a more preferred embodiment, the selection step is carried out at a pH of 4.5-6.5, at a temperature of 30-42°C, under at least partial anaerobiosis or under a combination of two or more of said conditions.

Any means for obtaining anaerobiotic conditions known in the art is suitable for the present invention. Anaerobiosis is usually achieved by increasing the partial pressure of a gas other than oxygen in the culture jar, like for instance, CO₂, such as in the AnaeroGen method manufactured by Oxoid, nitrogen or argon. In a more preferred embodiment, an extreme anaerobiosis is needed, in which case, the partial pressure of more than one gas has to be increased. An extreme anaerobiosis can be obtained the combined use of AnaeroGen and nitrogen and Anaerogen and argon. In a preferred embodiment, the extreme anaerobiosis is obtained by using a combination of AnaeroGen and nitrogen.

In a still more preferred embodiment, the method of the invention is carried out using a selection step at a pH of 5.8, at a temperature of 36°C and in a nitrogen/CO₂ atmosphere.

The culture medium and selection conditions have allowed for the first time to grow bifidobacteria present in human breast milk thanks to the use of a new culture media. Some of these bacterial strains exhibit a number of characteristics which render them beneficial to human health, and in particular in the prophylaxis or treatment against digestive, infective, neurodegenerative and other immune related diseases such as allergies or inflammatory diseases

Accordingly, in another aspect, the invention provides bacteria which are obtainable using the culture medium of the invention and the method of the invention. In a preferred embodiment, the bacteria obtainable using the culture medium of the invention is characterised in that it shows a bacillus morphology, it is gram(+), it shows fructose-6-phosphate phosphoketolase activity, it survives in the presence of 2% bile salts and is capable of fermenting galactose, D-glucose, D-fructose, esculine, maltose, melibiose, glycogen and D-turanose.

In a further preferred embodiment, the invention provides two strains which have been found as particularly useful for the above purpose and which have been deposited according to the Budapest Agreement at the CECT - Colección Española de Cultivos Tipo -, Valencia (Spain) on 17.04.2007 under accession numbers CECT N° 7263 and CECT under Accession N° 7264. Preferably, the strains deposited under CECT N° 7263 and CECT under Accession N° 7264 have been isolated using the selection method of the invention.

In another aspect, the invention provides a compositions which comprises at least one of the bacterial strains of the invention, either alone or comprising either another bacterial strain of the invention and/or another strain or mixture of strains, where each of the strains is present in the composition in a proportion from 0.1% to 99.9%, preferably from 1% to 99%, more preferably from 10% to 90%.

In a preferred embodiment, the invention also refers to compositions of the strains of this invention in a lyophilized form, freeze dried or inactivated (dead bacteria) by conventional methods.

In another aspect, the invention provides a foodstuff comprising a bacteria or a mixture of bacteria of the invention or a composition according to the invention. Non-limiting examples of suitable foodstuffs which can be used in the present invention are milk, yoghourt, cheese, curd, fermented milks, milk based fermented products, fermented cereal based products, fermented meat products, other milk based or cereal based powders, clinical nutrition formula, ice-creams, juices, bread, cakes or candies, animal feed formulations, semi- or synthetic diet formulations, infant formulae, clinical nutrition formulae, ice-creams, juices, flours, bread, cakes, candies or chewing-gums.

In another aspect, the invention provides a pharmaceutical composition comprising a therapeutically effective amount of at least a strain or a composition of the invention. The pharmaceutical preparation can take the form of tablets, capsules, liquid bacterial suspensions, dried oral supplements, wet oral supplements, dry tube feeding or a wet tube feeding.

The required dosage amount in the food or pharmaceutical composition described before will vary according to the nature of the disorder or the proposed use of the composition, whether used prophylactically or therapeutically and the type of organism involved. Therapeutic efficacy and toxicity may be determined by standard pharmaceutical procedures with experimental animals, such as by calculating the ED, (the dose therapeutically effective in 50% of the population) or LD (the dose lethal to 50% of the population) statistics. The dose ratio of toxic to therapeutic effects is the therapeutic index, which can be expressed as the LD/ED ratio. Nevertheless, the activity of the new microorganisms in the individual is naturally dose dependent. That is, the more the novel microorganisms are incorporated by means of ingesting or administration of the above food material or the pharmaceutical composition, the higher protective and/or therapeutic activity of the microorganisms. Since the microorganisms of this invention are not detrimental to mankind and animals and have eventually been isolated from healthy human breast milk, a high amount thereof may be incorporated so that essentially a high proportion of the individual's mucosa will be colonized by the novel microorganisms. Compositions which exhibit large therapeutic indices are preferred. The data obtained from animal studies are used to formulate a range of dosage for human or animal use. The dosage contained in such compositions is preferably within a range of circulating concentrations that includes the ED, with little or no toxicity. The dosage varies within this range depending upon the dosage form employed, the sensitivity of the patient, and the route of administration. The exact dosage will be determined by the practitioner, in light of factors related to the subject requiring treatment. For instance, for preparing a food composition according to the present invention at least one of the *Bifidobacterium* strains of the present invention is incorporated in a suitable support, in an amount of from 10⁵ cfu/g to about 10¹² cfu/g support material, preferably from about 10⁶ cfu/g to about 10¹¹ cfu/g support material, more preferably from about 10⁶ cfu/g to about 10¹⁰ cfu/g support material.

In the case of a pharmaceutical composition, the dosage of the probiotic strain should be from about 10⁵ cfu/g to about 10¹⁴ cfu/g support material, preferably from about 10⁶ cfu/g to about 10¹³ cfu/g support material, more preferably from about 10⁷ cfu/g to about 10¹² cfu/g support material. For the purpose of the present invention the abbreviation cfu shall designate a "colony forming unit" that is defined as the number of bacterial cells as revealed by microbiological counts on agar plates.

Dosage and administration are adjusted to provide sufficient levels of the active moiety or to maintain the desired effect. Factors which may be taken into account include the severity of the disease state, the general health of the subject, the age, weight, and gender of the subject, time and frequency of administration, drug combination(s), reaction sensitivities, and response to therapy. Long-acting compositions may be administered every 3 to 4 days, every week, or biweekly depending on the half-life and clearance rate of the particular formulation.

Preferably, the subject in need of treatment is selected from the group consisting of infants but also to other individuals who suffer the disorder or having risk to suffer the selected disorder, namely infection, allergy, inflammation, etc. However, it will be recognized that the present treatments are suitably employed in prophylaxis of those disorders in any subject.

Preferably the probiotic, or the probiotic-containing composition, is directed to the oral, gastric and/or to the intestinal mucosal surface; however, it could also be directed to nasopharyngeal, respiratory, reproductive or glandular mucosa, and/or to the mammary gland and it could be administered to human and animals by an oral, rectal, topical and/or vaginal route.

Further, the probiotics of the present invention may be used in conjunction with other treatments, to enhance or assist in their efficacy.

Many people have a disturbed intestinal microflora, that is, the balance between useful and harmful intestinal bacteria is disturbed. A number of factors, among others stress, the presence of bile salts, and specially diet, influence the bacterial flora. In these situations the fermentation process could be disturbed and the number of useful bacteria be reduced, the consequence would be that the colon mucosa withers away and ceases to function at the same time as the potentially malignant bacteria rapidly grow in number. For this reason, one aspect of this invention is the use of probiotics as prophylactic or therapeutic treatment of chronic or acute infection, or of undesirable microbial colonization, of a mucosal surface or any other human location, comprising the administration of an effective amount of a probiotic, or a probiotic-containing composition, to a subject in need thereof. In a preferred embodiment, the infection or colonization is caused by parasites, bacteria, yeast, fungi or viruses, of any body surface or mucosa in a subject or animal in need thereof.

The compositions of the present invention can also be used effectively in the treatment of acute and chronic viral infections. In particular the treatment of chronic Epstein-Barr virus, cytomegalovirus and other herpes-type virus infection, which are ubiquitous in the population and are associated with a decrease on the immune surveillance. Moreover, the compositions of the present invention can also be used for the treatment and prophylaxis of other more common acute or chronic viral infections of the intestinal and respiratory tract such as, but not restricted to rotavirus or influenza.

In a further aspect, the invention provides the use of the strain and compositions of the invention in the manufacture of a medicament for the treatment and/or prophylaxis of temporally depressed immune levels in individuals such as produced during aging or in healthy individuals who are subject to intense exertion or in general to a great physiological strain or stress.

In a further aspect, the invention provides the use of the strain and compositions of the invention in the manufacture of a medicament for the improvement of the immune gut barrier in a subject or animal in need thereof; for the treatment and/or prophylaxis of down-regulating hypersensitivity reactions to food and metabolic intolerance such as lactose intolerance; of constipation and other gastro-intestinal disorders. In another embodiment, the invention comprises the use of the probiotics of the invention for the prophylactic or therapeutic treatment of diarrhea, independently whether this disorder is due to the presence of a bacterial or viral infection, the treatment with antibiotics or chemo- or radiotherapy or to dietary or physical complications.

The probiotic strains are known to reduce the production of pro-inflammatory cytokines by activated macrophages known to chronic inflammatory disorders. Accordingly, in another aspect, the invention provides the use of the bacterial and compositions of the invention for the manufacture of a medicament for the treatment of inflammatory or auto-immune disorders. Non-limiting examples of such inflammatory and autoimmune diseases include IBD, ulcerative colitis, arthritis, atherosclerosis, multiple sclerosis, psoriasis or sarcoidosis.

Lactic acid bacteria are known to be useful for counteracting cancerdue to their effects in the inhibition of carcinogenic toxins in the intestines such as nitroseamines but also for the effect of this probiotics in the modulation of the natural immune response. Accordingly, in a further aspect, the invention provides the use of the strains stated in this invention for the prophylactic or therapeutic treatment of some cancer types and for inhibiting tumor growth, metastasis and cancer in subject or animal in need thereof.

The strains of the invention are capable of modulating the immune response and the balance between Th1 and Th2 cytokines. Accordingly, in a further aspect, the invention provides the use of the strain and compositions of the invention in the manufacture of a medicament for the treatment and/or prophylaxis of allergic disorders, asthma and disorders related with the development of tolerance against ingested proteins. Moreover, the strains described in the present invention are known to show hypocholesterolemic effects and to regulate the modulation of the oxidative stress effect of some strains of probiotics. Both situations have been related as risk factors for the development of neurodegenerative diseases. Moreover, it has also been described that commensal bacteria are able to deaminate L-tryptophan producing indole-3-propionic acid which is a potent neuroprotective agent. Accordingly, in still a further aspect, the invention provides the use of the strain and compositions of the invention in the manufacture of a medicament for the treatment and/or prophylaxis of neurodegenerative diseases in an individual or animal in need thereof. Neurodegenerative diseases that can be treated with the strains and compositions of the invention include, without being restricted to, the group consisting of Parkinson, stroke, Alzheimer, Huntington and dementia.

In still a further aspect, the invention provides the use of the strain and compositions of the invention in the manufacture of a medicament for the treatment and/or prophylaxis of metabolic and body weight control disorders.

Moreover, due to the presence of the selected strains in breast milk, the subjects in need of treatment could be not only those who intake directly the selected strains but also the fetus or breast feeding babies. Accordingly, in still a further aspect, the invention provides the use of the strain and compositions of the invention in the manufacture of a medicament designed to be administered to lactating woman for the therapeutic or prophylactic treatment of their fetus and/or their breastfed babies.

The following methods and examples illustrate the invention.

### EXAMPLES

### Example 1: Isolation of Bifidobacteria from breast milk

### Molecular detection of bifidobacteria in breast milk.

Breast milk samples from 23 healthy women were obtained 6-14 days after labor. None of the women had complications during labor and no antibiotic therapy was required. Each sample was initially checked for the presence of bifidobacteria by Real time PCR and PCR-DGGE.

The bands obtained by DGGE were subsequently sequenced demonstrating the existence of *B. breve, B. longum* and/or *B. adolescentis* in most of the milk samples. RT-PCR demonstrates that from all bacteria present in milk, the percentage of members of the *Bifidobacterium* genera oscillate between 0 and 16.05%. Although we were able to find bifidobacteria in 17 of 23 samples by molecular mechanisms, conventional platting of these milk samples in MRS supplemented with cysteine or other previously described supplements did not show similar results.

Conventional platting of the breast milk samples showed that only 3 of 23 samples contain cultivable bifidobacteria, but the colonies obtained were not able to growth in liquid media.

For these reasons we have decided to modify the culture media and the culture conditions in order to be able to isolate breast milk derived bifidobacteria ant to expand and growth these potential isolates.

### Selection of new culture conditions.

In order to evaluate potential new selection media for the growth of bifidobacteria present in the breast milk, we have prepared more than 50 different media. 100 µl of each breast milk sample was cultivated in each agar media. The different media were mainly composed of glucose, yeast extract, casein peptone, milk proteins, tween 80, several sources of essential fatty acids, different sources of fibre, and vitamins and salts.

From all these different culture media we selected the media M56, which composition is described in Table 2, since it was the media that allow us to obtain the higher number of positive milk samples (18 of 23) and the higher number of cultivable *Bifidobacterium* isolates. All isolates obtained were checked to belong to the *Bifidobacterium* genera by optical microscopy and 16 RNA sequencing.

Although M56 media demonstrated to be a good media for the isolation and expansion of breast milk-derived bifidobacteria, the growth rate was still quite slow. For this reason we decided to also modify the culture conditions for the propagation of these bacteria. Initially we used to growth the bifidobacteria at 37°C in anaerobiosis using the AnaeroGen from Oxoid obtaining values close to 2x10⁹ cfu/ml at 48 hours in M56 media at pH 6.2.

**Table 2: Composition of the M56 media*.**

| **Component** | **Concentration (g/l)** |
|---|---|
| Peptone | 10 |
| Goat casein | 5 |
| Glucose | 15 |
| Yeast extract | 2 |
| Tween 80 | 2 |
| Sodium acetate | 5 |
| Magnesium sulphate | 0.2 |
| Tri-amonium citrate | 2 |
| Olive oil | 2 |
| Fish oil | 0.5 |
| Rice bran extract | 1 |
| Fructo-oligosaccharides | 5 |
| Dicloxacilin | 0.0005 |
| Lithium chloride | 1 |
| Ascorbic acid | 0.5 |
| L-cysteine hydrochloride | 0.5 |

To prepare agar plates of this media we added 15 g/l of agar.

We have tested the effect to modify the temperature, pH and anaerobic conditions of the culture. In this sense we have tested the conditions described in Table 3. The growth curves obtained in this sense were showed in Figure 1.

**Table 3. Culture variables**

| **Conditions** | **Variables** |
|---|---|
| pH | free; 4.5; 5; 5.5; 5.8; 6; 6.2 and 6.5 |
| Temperature | 30°C, 32°C, 35°C, 36°C, 37°C, 38°C, 40°C, 42°C |
| Anaerobiosis | None |
| | AnaeroGen (Oxoid) |
| | Nitrogen |
| | Argon |
| | Nitrogen+AnaeroGen |
| | Argon+AnaeroGen |

From all these analysis we decided to fix the growth of breast-milk strains in M56 media at pH 5.8 and 36°C and incubated in extreme anaerobiosis (nitrogen+AnaeroGen) for 24-48 hours obtaining values higher than 8x10⁹ cfu/ml at 24 hours (Figure 1).

### Physiological and genetic characterization

Using this new media and the conditions described before, we were able to obtain 56 cultivable isolates belonging to the genera *Bifidobacterium.* All these isolates were physiological and genetically characterized and submitted to a screening process to evaluate the potential probiotic characteristic of the different bacteria and their potential health benefits.

From all the isolates we finally selected two of them due to their excellent probiotic properties (described in the following examples. The two selected bacteria were:
(iii) *Bifidobacterium breve* CECT 7263
(iv) *Bifidobacterium breve* CECT 7264
The 16S RNA sequence of these two bacteria and their rapid profile were showed in Figure 2. Moreover, Table 4 summarizes the physiological characteristics of these two bifidobacteria grown on M56 media (agar or broth) at 36°C in extremely anaerobic conditions, such as their morphology, gram staining, some enzymatic activities, the ability top produce gas or their resistance to bile salts.

**Table 4: Phenotypic characteristics of the different probiotic strains of the invention.**

| **Characteristic** | ***B. breve* CECT 7263** | ***B. breve* CECT 7264** |
|---|---|---|
| Morphology | bacillus | bacillus |
| Gram staining | + | + |
| Catalase | - | - |
| Oxidase | - | - |
| Nitrate reductase | - | - |
| Gelatinase | - | - |
| F6PPK | + | + |
| Indol Production | - | - |
| Gas Production | - | - |
| Arabinose degradation | - | - |
| Survival to 2% Bile salts | + | + |

Moreover, we performed a fermentation API 50CH (BioMerieux) analysis of the probiotic strains belonging to this invention at 36°C in extreme anaerobic conditions for 24 and 48 hours and following the specified instructions indicated by the manufacturer. The 24 hours results were summarized in Table 5. A positive fermentable substrate is that with a value higher than 3.

Due to the low fidelity of API characterization, we also performed the analysis of the 16S RNA sequence of the selected bacterial strains. The sequences obtained were showed in Figure 2 besides the PCR-RAPD profile of the strains.

**Table 5: Fermentation pattern of the different probiotic strains of the invention**

| TEST | 7263 | 7264 |
|---|---|---|
| Glycerol | 0 | 0 |
| Erythritol | 0 | 0 |
| D-Arabirose | 0 | 0 |
| L-Arabinose | 0 | 0 |
| Ribose | 5 | 0 |
| D-Xylose | 0 | 0 |
| L-Xylose | 0 | 0 |
| Adonitol | 0 | 0 |
| Methyl-xyloside | 0 | 0 |
| Galactose | 5 | 5 |
| D-Glucose | 5 | 5 |
| D-Fructose | 5 | 5 |
| D-Mannose | 1 | 0 |
| L-Sorbose | 0 | 0 |
| Rhamnose | 0 | 0 |
| Dulcitol | 0 | 0 |
| Inositol | 0 | 0 |
| Mannitol | 4 | 0 |
| Sorbitol | 5 | 0 |
| Methyl-D-mannoside | 0 | 0 |
| Methyl-D-glucoside | 2 | 0 |
| N Acethyl glucosamine | 2 | 1 |
| Amygdaline | 0 | 1 |
| Arbutine | 0 | 0 |
| Esculine | 5 | 5 |
| Salicine | 1 | 0 |
| Cellobiose | 5 | 0 |
| Maltose | 5 | 5 |
| Lactose | 5 | 0 |
| Melibiose | 5 | 5 |
| Saccharose | 5 | 0 |
| Trehalose | 0 | 0 |
| Inuline | 0 | 0 |
| Melezitose | 0 | 0 |
| D-Raffinose | 5 | 0 |
| Amidon | 5 | 0 |
| Glycogene | 5 | 4 |
| Xylitol | 0 | 0 |
| Gentiobiose | 0 | 2 |
| D-Turanose | 5 | 4 |
| D-Lyxose | 0 | 0 |
| D-Tagatose | 0 | 0 |
| D-Fucose | 0 | 0 |
| L-Fucose | 0 | 0 |
| D-Arabitol | 0 | 0 |
| L-Arabitol | 0 | 0 |
| Gluconate | 0 | 0 |
| 2 ceto-gluconate | 0 | 0 |
| 5 ceto-gluconate | 0 | 0 |

The results obtained for all these tests lead to the classification of the bacterial strains as indicated above. With this classification the bacterial strains of the invention were deposited according to the Budapest Agreement at the CECT- *Colección Española de Cultivos Tipo-,* Valencia (Spain) on April, 17th, 2007 and with the following accession numbers:
(i) *Bifidobacterium breve* CECT 7263
(ii) *Bifidobacterium breve* CECT 7264

### Example 2: Probiotic screening of the strains

Most of the selected strains were analyzed or screened for a high number of different characteristics that could enhance their capabilities to act as a probiotic strains. The results obtained are described in the indicated examples.

### Culture of Caco-2 and HT-29 cells

For the adhesion and inhibition assays the cell lines Caco-2 (ATCC HTB-37) and HT-29 (ATCC HTB-38) were utilized as a model of the intestine cells. Both cell lines presented features characteristic for intestinal cells such as polarization, expression of intestinal enzymes, production of particular structural polypeptides and mucins.

The cells were grown in plastic flasks (75 cm2, Nunc) in DMEM as culture medium supplemented with 10% inactivated FCS, non essential amino acids, 100 U/ml penicilline/streptomycine, 1 µg/ml amphoterine. Culturing was performed at 37°C in an atmosphere comprising 95% air and 5% CO₂. Media was changed on a two daily basis and the cells were splitted every week.

For the adhesion assays the cells were splitted to 35 mm plastic dishes (Nunc) and cultured in similar conditions but without antibiotics after confluence. Adhesion assays were performed 10-14 days post-confluence.

### Culture of bacteria

### (iii) Probiotic strains:

All probiotic strains of this invention were cultured in M56 broth (pH 5.8) in extreme anaerobic conditions for 24 hours at 36°C after inoculation of a 1 % (v/v) from the FOS-olive-glycerol stock. At these conditions, the concentration of the culture was 5-8x10⁹ cfu/ml, as observed by plating on M56 agar.

### (iv) Gram-negative strains:

*Escherichia coli* O157:H7 (non-pathogenic) (CECT4972), *E.coli* O157:H7 (entero-pathogenic) (CECT4783), *E.coli* O157:H7 (entero-pathogenic) (CECT4782), *Salmonella cholerasuis typhi* (CECT409) and *S. cholerasuis typhimurium* (CECT443) were all obtained from the CECT*-Colección Española de Cultivos Tipo-.* All gram negatives strains were cultured in TSB broth (AES Laboratoire) in anaerobic conditions for 16-18 hours at 37°C after inoculation of a 0.1% (v/v) from the glycerol stock. At these conditions, the concentration of the culture was 1-2x10⁹ cfu/ml, as observed by plating on TSA agar (AES Laboratoire).

### Adhesion analysis to Caco-2 and HT-29

Caco-2 and HT-29 intestinal cell lines were cultured in 35 mm plastic dishes in 2 ml medium without antibiotics to confluence. 10-14 days post-confluence 1 ml of media was replaced with 1 ml of a suspension of 10⁸ bacteria in DMEM (PAA). The cultures were incubated 1 hour at 37°C. After that, cells were washed twice with PBS and fixed with ice-cold 70% methanol for 30 minutes. Plates were air dried and Gram stained. The attached bacteria were visualized using an optical Axiovert 200 (Zeiss) microscope at 1000x magnification in oil-immersion. Twenty randomized fields were counted and the results expressed as the mean of the number of bacteria attached to the cells per field ±SD (Figure 3)

### Resistance to acid and bile salts

To analyze the resistance of the probiotic strains of this invention to acidic and high bile salt content, conditions that these bacteria will encounter during the digestive transit, bacteria were cultured in M56 broth media pH 3.0 or with 2 % bile salts (Sigma) for 90 minutes. The survival was calculated by M56 agar plating of serial dilutions and compared to the number of colonies obtained in control conditions (M56 broth pH 5.8). Plates were cultured 24 hours at 36°C in extreme anaerobic conditions. The experiment was repeated three times (Figure 4).

### Fermentation capabilities

The ability of a bacterial strain to use complex carbohydrates (soluble and non-soluble fibers) ensures that these probiotic strains could use them as a source of carbohydrates in the colon since they are not digested, and thus enhance the efficiency of colonization. For this reason, we have tested the capability of the probiotic strains of this invention to use several fibers as an unique source of carbohydrates.

To assay the capability to ferment fiber we cultured the probiotic bacterial strains in M56 without carbohydrate source and supplemented with a 2% of each fiber for 24 and 48 hours at 36°C in extreme anaerobic conditions. This experiment was performed in 96 well flat-bottomed plastic dishes (Nunc). The fermentation process was assessed as the reduction of the pH in the media by a colorimetric approach using 0.3% phenol red as an indicator and measuring the absorbance at 540 nm.

The fibers used were: α-cellulose (raw cellulose, Campi y Jove), Actilight (fructo-oligosaccharide, Beghin-Meiji), Ficao (cocoa fiber, Natra), Fructafit (Inulin, Sensus), Lactose (Bordulo), Pectine (YM100, Genu), Raftiline (Inulin oligofructose, Orafti), Raftilose (Inulin oligofructose, Orafti), and Vitacel (purified cellulose, Campi y Jove) (Table 6)

**Table 6: Fermentation capabilities of the different probiotic strains of the invention**

| **Characteristic** | ***B. breve* CECT 7263** | ***B. breve* CECT 7264** |
|---|---|---|
| α-cellulose | +/- | +/- |
| FOS | +++ | +++ |
| Cocoa Fiber | + | ++ |
| Inulin | + | ++ |
| Lactulose | +++ | +++ |
| Pectine | ++ | +/- |
| Oligofructose | +++ | +++ |
| Purified cellulose | +/- | +/- |

### Resistance to antibiotics

The use of modern antibiotics leads to a reduction of the comensal gut microflora which sometimes relates to diarrhea and other gut disorders. Moreover, this reduction in the amount of gut bacteria could be the consequence of opportunistic pathogenic bacteria and viruses to infect the host. The use of antibiotics to block the infection does not resolve this disorder but complicates it. In other situations like intestinal inflammation where probiotics could exert a beneficial role, this potential effect is sometimes limited for the simultaneous therapy with antibiotics. For all these reasons, the selection of potential probiotic strains which were able to resist common antibiotics should be clearly interesting.

To analyze the resistance of the probiotic strains of this invention we used a agar well diffusion assay. Müeller-Hinton agar plates containing 10⁶ cfu/ml of each probiotic strain were prepared. Then, antibiotic commercial discs were added to the wells and allowed to diffuse into the agar during 10 minutes preincubation period at room temperature, followed by extreme anaerobic incubation of the plates at 36°C for 16-18 hours.

In this conditions both bacteria showed sensibility to penicillin, amoxicillin, clavulanic acid, ampiciline, nitrofurantoine, bacitracine, cefoxitine, tetracycline, erythromycin, espiromicine, clindamycin, vancomycin, chloramphenicol, and riphampicin; while both bifidobacteria were resistant only to low concentrations of kanamycin (30 µg) and gentamicine (15 µg).

### Production of bactericidal metabolites

It has been suggested that the main mechanism used by probiotics is controlling the balance between useful and harmful intestinal bacteria is the gut. When the number of useful bacteria is reduced, opportunistic bacteria could over-grow and disturb the well-being of the host or even induce an infection. Most bacterial organisms have acquired characteristics or mechanisms that reduce the growth capabilities of other microorganisms that cohabitate with them and thus, enabling their selective growth. The reduction of pH through acid production by lactic acid bacteria is one of such mechanisms. Moreover, some lactic bacteria also produce bioactive peptides components and other metabolites that selective inhibit the growth of other bacteria, yeast or fungi. This is the case of bacteriocins such as e.g. reuterin or pediocin.

The probiotic strains of this invention were assessed for their capability to produce bactericidal metabolites using an agar well diffusion assay. TSA agar plates containing 10⁶ cfu/ml of different pathogenic bacteria strain *(S. typhi, S. typhimurium and E.coli)* were prepared. Wells, with a diameter of 5 mm, where then cut in the agar using a sterile cork-borer. Then, 50 µl of a 2 fold concentrate supernatant of each probiotic strain solution corresponding to the indicated concentrations were added to the wells and allowed to diffuse into the agar during a 2 hours preincubation period at 4°C, followed by aerobic incubation of the plates at 37°C for 16-18 hours (Figure 5).

### Inhibition of pathogen adhesion to Caco-2

Caco-2 intestinal cell lines were cultured in 35 mm plastic dishes in 2 ml medium without antibiotics to confluence. 10-14 days post-confluence 1 ml of media was replaced with 1 ml of a suspension of 10⁸ probiotic bacteria in DMEM. The cultures were incubated 1 hour at 37°C. After that, 1 ml of a suspension of 10⁸ pathogenic bacteria (*E. coli* or *S. typhimurium*) in DMEM was added to the cultures and incubated 1 hour more at 37°C. The cells were washed twice with PBS and fixed with ice-cold 70% methanol for 30 minutes. Plates were air dried and Gram stained. The attached bacteria were visualized using an optical Axiovert 200 (Zeiss) microscope at 1000x magnification in oil-immersion. The number of gram-negative bacteria in 10 randomized fields were counted and the results expressed as the mean of % of pathogenic bacteria attached to the cells compared to control cultures without probiotic strains (Figure 6).

### Probiotic colonization of mice gut

By definition a probiotic must colonize the gut mucosa of the host. Moreover, it has been described that the beneficial actions exerted by probiotics require this colonization. Although *in vitro* studies such as adhesion capabilities to intestinal cell lines, or resistance to the digestion conditions are good approaches to select probiotic strains, these tests do not ensure the effectiveness of the selected strain to *colonize in vivo* the gut mucosa. For this reason we performed an *in vivo* analysis of the capacity of the probiotic strains of the invention using mouse as an experimental animal model.

Male Balb/c mice (6-8 weeks old) were daily supplemented with 10⁸ cfu in 0.2 ml of skimmed milk of *B. breve* CECT 7263 or CECT 7264 for 14 days. After this period, the probiotic supplementation was finalized but the animals still in observation for another 14 days. Feces samples were collected at 0, 7, 14, 21 and 28 days from the initiation of the experience. Approximately 200 mg of feces were collected independently from each mice and homogenized at a 50 mg/ml in peptone water. The collected supernatant was serial diluted, and 0, 1 ml cultured in selective agar plates (MRS for Lactobacilli, Eugon agar + tomato juice for Bifidobacteria and McConkey agar for coliform bacteria) for bacterial counting. Plates were incubated at 37°C in anaerobic conditions for 24 hours.

Figure 7 shows that supplementation with *B. breve* CECT 7263 or CECT 7264 was able to increase the number of total bifidobacteria in feces which demonstrates that this strain is able to survive its passage through the digestive tract and arrive to the colon. Moreover, the fact that the increased bifidobacteria count was still observable one week after finalization of the oral supplementation demonstrates that this probiotic strain is able to temporally colonize the gut mucosa.

Interestingly, together with the bifidobacteria increase we observed also an increase in lactobacilli numbers and a reduction in the fecal count of coliform bacteria, which was still evident two weeks after finalization of the probiotic treatment.

### Example 4: Effect of B. breve CECT 7263 or CECT 7264 on translocation of Salmonella typhimurium in mice following immunization with inactivated Salmonella vaccine

Translocation of gram-negative bacteria across the gut epithelium can occur especially in subjects following gastrointestinal infection, disease or surgery. Left untreated it can lead to endotoxemia. In this example, the effect of feeding *B. breve* CECT 7263 or CECT 7264 on the translocation of gut pathogen *Salmonella typhimurium* was examined.

Male Balb/c mice (6-8 weeks old) were daily fasted with 1x10⁸ cfu in 0.2 ml of milk or milk alone for two weeks. After that, mice were immunized orally or not with an inactivated *Salmonella* vaccine (10⁸ cfu inactivated with paraphormaldehyde in 0.2 ml milk). After immunization, mice were fasted two weeks more with the probiotic preparations in alternate days for two weeks more. Two weeks after oral immunization, all mice were orally challenged with live *S. typhimurium* (10¹⁰ cfu in 0.2 ml milk). Then, after 24-48 hours, the level of colonization of *S. typhimurium* in the spleen was determined. We also analyzed the levels of IgA specific for *Salmonella* antigens in feces.

The results obtained demonstrate that *B. breve* CECT 7263 and CECT 7264 potentiates the beneficial effect of the vaccination of mice with the inactivated *Salmonella* vaccine as shown in Figure 8. The inhibition on the translocation of *S. typhimurium* induced by the inactivated vaccine and potentiated by *B. breve* CECT 7263 and CECT 7264 was due to the increase of the secretion of specific IgA and also, probably, to the inhibition or blocking effect of the probiotic strain on the mucosal adhesion of *Salmonella* as described in Example 2h.

### Example 5: Effect of probiotic bacteria on inflammatory cytokines

Besides the reduction of the risk of infection, many clinical effects associated to probiotic treatments are due to immunomodulatory capabilities of selected probiotic strains. The regulation of the immune response is usually mediated through a change in the balance between pro-inflammatory cytokines (Th1) such as TNF-α, humoral cytokines (Th2) such as IL-4 or IL-13, and regulatory cytokines (Th3) such as IL-10 and TGF-β. For this reason, we have also tested the effect of some of the probiotic strains of this invention in regulating the expression of some of these clue cytokines. We have used bone marrow derived macrophages stimulated with 100 ng/ml of LPS (Sigma) as a cellular model. 10⁵ macrophages/well were cultured in 24-well plastic plates (Nunc) with 1 ml of DMEM. Once attached, macrophages were stimulated or not with 100 ng/ml LPS and with 10⁷ cfu/ml of the indicated probiotic strains for 12 hours at 37°C in a 5% CO₂ atmosphere. Supernatants were collected and the production of cytokines was analyzed using a mouse TNF-α or mouse IL-10 ELISA (Biosource).The results obtained are summarized in Figure 9.

### Example 6: Effect of probiotic bacteria on Ig production

The analysis of the effect of the probiotic strains of this invention on the Immunoglobulin production was performed using lymphocyte cultures obtained from the spleen of male Balb/c mice (6-8 weeks old). 2×06 lymphocytes were cultured in 1 ml DMEM in 24 well plastic plates and stimulated with inactivated probiotic cultures (10⁸ cfu/ml) in presence or absence of 25 µg/ml LPS for 6 days. The production of Ig G by lymphocytes was assessed using a mouse Ig G ELISA from Bethyl (Figure 10).

### Example 7: Preparation of a fermented liquid milk formula

A normal fermented liquid milk composition with probiotics was prepared using the following formula:

| | |
|---|---|
| Milk 1.5% Fat; 3.2% protein | 997 g/Kg |
| Skim milk powder | 3 g/Kg |
| Probiotic strain (10¹² cfu/g) | 0.1 g/Kg |

### Processing technology:

- Milk standardization. The fat and dry solids contents of the milk are normally standardized according to the formulation described before.
- Homogenization. The milk was homogenized at 20-25 Mpa and 65-70°C to obtain optimum physical properties in the product.
- Heat treatment. Heat treatment was performed at 90-95°C and a holding time of about 5 minutes which is able to denature about 70-80% of whey proteins.
- Fermentation. Cooled milk (40-45°C) was inoculated with the probiotic strain in absence of starter culture and fermented in the incubation tank at 40-45°C for about 10 hours without agitation until reaching the final pH (pH 4.5-5).
- Homogenization. After clod formation, it is homogenized by mechanical methods.
- Cooling. The cooling was performed very quickly obtaining a temperature below 10°C in 60 minutes. After that, the composition is packaged. Final cooling, normally down to 5°C, takes place in a cold room, where the products are held to await distribution.

### Example 10: Preparation of a set yogurts

A normal yogurt product with probiotics was prepared using the following formula:

| | |
|---|---|
| Milk 3.1 % Fat; 3.2% protein | 987 g/Kg |
| Skim milk powder | 13 g/Kg |
| Starter | 0.1 g/Kg |
| Probiotic strain (10¹² cfu/g) | 0.1 g/Kg |

### Processing technology:

- Milk standardization. The fat and dry solids contents of the milk are normally standardized according to the formulation described before.
- Homogenization. The milk was homogenized at 20-25 Mpa and 65-70°C to obtain optimum physical properties in the product.
- Heat treatment. Heat treatment was performed at 90-95^{a}C and a holding time of about 5 minutes which is able to denature about 70-80% of whey proteins.
- Packaging. After pasteurization, milk was cooled to 40-45°C and the starter and probiotic cultures were metered into the stream of milk as they were pumped from an intermediate storage tank to the filling machine. Following packaging in the filling machine, the packages after crating and palletizing, are trucked into the system for incubation and cooling.
- Fermentation. Filled pallets were fermented in the incubation room at 40-45°C for about 5-6 hours until a pH of 4.5 is reached.
- Cooling. The cooling of the packets were performed quickly obtaining a temperature of 12-15°C in 55-70 minutes. Final cooling, normally down to 5°C, takes place in the chill store, where the products are held to await distribution.

### Example 11: Preparation of an infant formula in powder

An infant formula with probiotics was prepared using the following formula:

| | |
|---|---|
| Demineralised whey | 512 g/Kg |
| Palm olein | 135 g/Kg |
| Lactose | 92 g/Kg |
| Skimmed Milk | 95 g/Kg |
| Rapeseed oil | 52 g/Kg |
| Coconut oil | 49 g/Kg |
| Sunflower oil | 28 g/Kg |
| Water | 31 g/Kg |
| Vitamin premix | 2 g/Kg |
| Mineral premix | 4 g/Kg |
| Probiotic strain (10¹² cfu/g) | 0.1 g/Kg |

### Processing technology

- Milk standardization to an appropriately sized blend tank with agitation and heating all solid ingredients were mixed with the liquid milk and water in the absence of any vitamins. Then, the vegetable oils were admixed.
- Homogenization. The mixture was then heated at 60-70° C and emulsified through a single stage homogenizer at 6 to 7 MPa in absence of oxygen.
- Addition of vitamins. After emulsification the mixture was standardized by addition of vitamins and the pH was adjusted in the range of about 6.7 to 7.2.
- Heat treatment. The mixture was reheated to between about 65°C and 70°C.
- Spray drying: The product was finally was dried in a spray drier to obtain a final dry powder product.
- Addition of the probiotic strain. The Probiotic strain (10¹² cfu/g) (0.1g/Kg) was dry mixed with the final dry powder product and was packaged.

## Claims

1. A lactic acid bacteria culture media supplemented with olive oil, fish oil, ascorbic acid, rice bran extract, fructo-oligosaccharides and goat casein.

2. A culture media according to claim 1 which comprises peptone, goat casein, glucose, yeast extract, Tween 80, sodium acetate, magnesium sulfate, triammonium citrate, olive oil, fish oil, rice bran extract, fructo-oligosaccharides, dicloxacilin, lithium chloride, ascorbic acid and L-cysteine hydrochloride.

3. A culture media according to claim 2 wherein the concentrations in g/l of the different components are
| | |
|---|---|
| Peptone | 10 |
| Goat casein | 5 |
| Glucose | 15 |
| Yeast extract | 2 |
| Tween 80 | 2 |
| Sodium acetate | 5 |
| Magnesium sulfate | 0.2 |
| Tri-ammonium citrate | 2 |
| Olive oil | 2 |
| Fish oil | 0.5 |
| Rice bran extract | 1 |
| Fructo-oligosaccharides | 5 |
| Dicloxacilin | 0.0005 |
| Lithium chloride | 1 |
| Ascorbic acid | 0.5 |
| L-cysteine hydrochloride | 0.5 |

4. Use of a culture media according to claims 1 to 3 as a media to grow probiotic bacteria.

5. Method for the isolation of probiotic bacteria comprising the steps of
(i) Incubating a sample suspected of containing a probiotic strain in a culture medium according to claims 1 to 3 and
(ii) selecting strains which are capable of growing in said culture medium.

6. Method according to claim 5 wherein the sample suspected of containing a probiotic strain is human breast milk.

7. Method according to claims 5 or 6 wherein the selection step is carried out at a pH of 4.5-6.5, at a temperature of 30-42°C, under at least partial anaerobiosis or under a combination of more than one of said conditions.

8. Method according to claims 5 to 7 wherein the selection step is carried out at a pH of 5.8, at a temperature of 36°C and in a nitrogen/CO₂ atmosphere.

9. A bacterial strain obtainable by the method according to claims 5 to 8.

10. A bacterial strain according to claim 9 **characterised in that** it shows a bacillus morphology, it is gram(+), it shows fructose-6-phosphate phosphoketolase activity, it survives in the presence of 2% bile salts and is capable of fermenting galactose, D-glucose, D-fructose, esculine, maltose, melibiose, glycogen and D-turanose.

11. A bacterial strain deposited in the CECT under Accession N° 7263

12. A bacterial strain deposited in the CECT under Accession N° 7264.

13. A composition comprising at least one of the bacterial strains specified in claims 9 to 12.

14. A composition according to claim 13 further comprising another strain or mixture of strains.

15. A composition according to claims 12 or 13 which is in lyophilized fonn or frozen fonn.

16. A composition according to claims 13 to 15 in an inactivated (dead) form.

17. Food or animal feed product comprising at least one strain according to claims 9 to 12 or a composition according to claims 13 to 16.

18. A pharmaceutical product comprising a therapeutically effective dose of at least one strain according to claims 9 to 12 or a composition according to claims 13 to 16.

19. A strain according to claims 9 to 12 or a composition according to claims 13 to 16 for therapeutic or prophylactic treatment of human and animal diseases.

20. Use of a strain according to claims 9 to 12 or a composition according to claims 13 to 16 in the manufacture of a medicament for the therapeutic or prophylactic treatment of chronic or acute infection or of undesirable microbial colonization of any body surface or mucosa in a subject or animal in need thereof.

21. Use of a strain according to claims 9 to 12 or a composition according to claims 13 to 16 in the manufacture of a medicament for the treatment and/or prophylaxis of temporally depressed immune levels in a subject or animal in need thereof.

22. Use of a strain according to claims 9 to 12 or a composition according to claims 13 to 16 in the manufacture of a medicament for the improvement of the immune gut barrier in a subject or animal in need thereof; for the treatment and/or prophylaxis of down-regulating hypersensitivity reactions to food and metabolic intolerance such as lactose intolerance; of constipation and other gastro-intestinal disorders; of inflammatory or auto-immune disorders such as: IBD, ulcerative colitis, arthritis, atherosclerosis, multiple sclerosis, psoriasis or sarcoidosis; and of tumor growth, metastasis and cancer in subject or animal in need thereof.

23. Use of a strain according to claims 9 to 12 or a composition according to claims 13 to 16 in the manufacture of a medicament for the treatment and/or prophylaxis of allergic disorders and asthma in a subject or animal in need thereof.

24. Use of a strain according to claims 9 to 12 or a composition according to claims 13 to 16 in the manufacture of a medicament for the treatment and/or prophylaxis of neurodegenerative diseases in an individual or animal in need thereof.

25. Use of a strain according to claims 9 to 12 or a composition according to claims 13 to 16 in the manufacture of a medicament for the treatment and/or prophylaxis of metabolic and body weight control disorders.

26. Use of a strain according to claims 9 to 12 or a composition according to claims 13 to 16 in the manufacture of a medicament designed to be administered to lactating woman for the therapeutic or prophylactic treatment of their fetus and/or their breastfed babies.
